# EUROPÄISCHE PATENTSCHRIFT

(11) **EP 0 477 551 B1**
(45) Veröffentlichungstag und Bekanntmachung des Hinweises auf die Patenterteilung: **25.01.1995**
(21) Anmeldenummer: 91114320.4
(22) Anmeldetag: 27.08.1991
(51) Int. Cl.: A61G 12/00, H05K 7/14, A61M 5/142

(54) **Infusionsgeräteträger**
Support for infusion apparatus
Support pour appareilles de pérfusion

(30) Priorität: 26.09.1990 DE 4030368
(43) Veröffentlichungstag der Anmeldung: 01.04.1992
(73) Patentinhaber: B. Braun Melsungen AG, 34209 Melsungen (DE)
(72) Erfinder: Heitmeier, Rolf, W-3508 Baunatal (DE); Proell, Dieter, W-3501 Körle (DE)
(74) Vertreter: Selting, Günther, Dipl.-Ing.

(56) Entgegenhaltungen:
- DE-A- 3 603 728
- DE-U- 8 910 111
- GB-A- 2 133 973
- US-A- 4 756 706

## Beschreibung

Die Erfindung betrifft eine Infusionsvorrichtung der im Oberbegriff des Patentanspruchs 1 angegebenen und aus DE 36 03 728 A1 bekannten Art.

Die Entwicklung in der Intensivmedizin hat zu Infusionstherapien geführt, die den gezielten Einsatz hochwirksamer Medikamente erfordern. Je nach benötigter Wirkung werden, basierend auf dem diagnostizierten Krankheitsbild, bis zu zwölf Medikamente parallel infundiert. Hinzu kommen eventuell parenterale Ernährungen, bei denen große Volumenmengen pro Zeiteinheit dem Patienten zugeführt werden. In der Summe können somit am Bett einer Intensivstation bis zu 16 Infusionsgeräte zum Einsatz kommen.

Neben der Notwendigkeit der Durchführung der Therapie besteht die Notwendigkeit, die Infusionstherapie zu dokumentieren und systemübergreifende Therapiedaten zu präsentieren bzw. für den Anwender in sinnvoller Form, z.B. als Grafik, anschaulich darzustellen. Dies wird durch Dokumentationssysteme ermöglicht, die sich als "Bedside-Rechner" realisieren lassen. Hierzu sind jedoch elektrische Verbindungen der einzelnen Infusionsgeräte mit dem Rechner erforderlich, die eine umfangreiche und unübersichtliche Verdrahtung erfordern.

Bekannt sind Infusionsgeräte, die mehrere Infusionspumpen enthalten. Hierbei ist die Zahl der Infusionspumpen durch die Konstruktion des Gerätes begrenzt und der technische Aufwand ist hoch, weil das Gerät mit der maximalen Zahl von Infusionspumpen ausgerüstet ist, von denen jedoch nicht immer alle benutzt werden. Darüber hinaus haben derartige Einrichtungen keine Eingriffsmöglichkeit für den Anwender, wenn die zentrale Steuerung ausfällt. Derartige Kompaktlösungen können also nicht einer sich ändernden Infusionstherapie angepaßt werden. Ihre Flexibilität ist für den Anwender stark eingeschränkt. Hinzu kommt die mangelnde Sicherheit derartiger Geräte. Bei Ausfall der zentralen Steuerung fallen sämtliche Infusionspumpen aus.

Die bekannte Infusionsvorrichtung nach DE 36 03 728 A1 weist eine an einer Wand zu befestigende Hohlprofilschiene auf, an der mehrere Pumpenträger zur Aufnahme jeweils eines Infusionsgerätes befestigt sind. Die Pumpenträger sind mit dem Boden des Infusionsgerätes über Löcher verschraubt. Sie werden ihrerseits mit Rändelschrauben an der Hohlprofilschiene leicht lösbar befestigt. Beim Abnehmen der Infusionspumpe von der Hohlprofilschiene verbleibt der Pumpenträger am Gehäuse der Infusionspumpe. Nach Anbringung einer mit einem Pumpenträger versehenen Infusionspumpe an der Hohlprofilschiene müssen noch die elektrischen Verbindungen hergestellt werden, indem die Netzstecker und Alarmstecker der Infusionspumpen in die entsprechenden Steckdosen der Hohlprofilschiene eingesteckt werden. Dies führt bei mehreren Infusionspumpen schnell zu einer unübersichtlichen Kabelführung, wobei nicht erkennbar ist, welches der Infusionsgeräte ordnungsgemäß angeschlossen ist.

Aus DE 34 02 885 A1 ist eine Versorgungssäule für medizinische Zwecke bekannt, die auch Infusionsgeräte tragen kann. Diese Säule weist vertikale Schienen auf, in denen Konsolen, Halter und Zubehörteile verschiebbar und mittels Gleitverriegelungen arretierbar sind. Die Säule enthält elektrische Leitungen für Netzspannung, Niedrigspannung, Notversorgung, Erde usw. und Fluidleitungen für Gas, Luft, Sauerstoff und Vakuum sowie die entsprechenden Anschlüsse oder Auslässe. Eine solche Versorgungssäule ermöglicht zwar eine geordnete Anbringung von Infusionsgeräten, jedoch verwirren sich die Netzkabel und Datenkabel mehrerer Infusionsgeräte auch hier zu einem unübersichtlichen Wirrwarr.

Eine Versorgungseinheit für eine medizinische Pflegestation ist schließlich noch bekannt aus DE 35 33 229 A1. Hierbei ist ein Versorgungsbalken vorgesehen, der als Träger für verschiedene medizinische Geräte dient und elektrische Leitungen und Anschlußelemente für diese Geräte aufweist.

Der Erfindung liegt die Aufgabe zugrunde, eine Infusionsvorrichtung zu schaffen, die nicht nur ein sinnvolles Ordnungs- und Plazierungssystem für unabhängig arbeitende Infusionsgeräte bildet, sondern auch die Anbringung der Infusionsgeräte und ihren elektrischen Anschluß erleichtert.

Die Lösung dieser Aufgabe erfolgt erfindungsgemäß mit den Merkmalen des Patentanspruchs 1.

Bei der erfindungsgemäßen Infusionsvorrichtung ist der Geräteträger als elektrische Versorgungseinheit für die Infusionsgeräte ausgebildet. An dem Geräteträger werden die einzelnen Infusionsgeräte als aufsteckbare Einheiten befestigt. Bei der Befestigung der Infusionsgeräte wird zugleich über den Geräteträger deren elektrische Stromversorgung über Kopplungselemente des Geräteträgers sichergestellt. Die einzelnen Infusionsgeräte haben mit den Kopplungselementen des Geräteträgers zusammenpassende Gegenstücke, so daß separate Versorgungskabel nicht erforderlich sind. Es entfallen daher unübersichtliche Kabelverlegungen. Wenn ein Infusionsgerät in der richtigen Lage am Geräteträger befestigt ist, ist zugleich die Stromversorgung des Infusionsgerätes sichergestellt. Der Anwender braucht also nicht zu kontrollieren, ob ein Netzanschluß hergestellt ist.

Die Kopplungselemente sind zweckmäßigerweise nicht nur für die Stromversorgung vorgesehen, sondern auch für die Verbindung von Datenanschlüssen der Infusionsgeräte mit Datenleitungen des Geräteträgers. Es ist daher möglich, den Geräteträger mit einer zentralen Überwachungs- und/oder-Steuereinrichtung auszustatten oder eine solche Vorrichtung an den Geräteträger anzuschließen. Damit kann der Betrieb sämtlicher angeschlossener Infusionsgeräte zentral überwacht, gesteuert und ggf. dokumentiert werden. Die Kopplungselemente sind im einfachsten Fall Steckverbinder, durch die die Verbindung der Infusionsgeräte mit dem Geräteträger sichergestellt wird. Anstelle mechanischer Steckverbinder können aber auch andere Kopplungselemente benutzt werden, beispielsweise für die Energieversorgung induktive Kopplungselemente und für die Datenübertragung optische oder induktive Kopplungselemente. Derartige Kopplungselemente haben den Vorteil, daß sie keine bewegbaren mechanischen Teile benötigen und leicht zu desinfizieren sind.

Gemäß einer bevorzugten Ausführungsform der Erfindung weist der Geräteträger eine Säule auf, an die die Infusionsgeräte derart angesteckt werden können, daß sie die Säule von drei Seiten her umgeben. Die Infusionsgeräte sind als Moduleinheiten ausgebildet, die einzeln an der Säule befestigt werden können. Dabei ist es nicht erforderlich, daß sämtliche Geräteplätze der Säule mit einem Infusionsgerät bestückt sind. Es werden nur jeweils soviele Infusionsgeräte an die Säule angesetzt wie für die Therapie erforderlich sind. Die übrigen Infusionsgeräte können anderweitig in Verbindung mit anderen Geräteträgern oder auch separat benutzt werden.

Für die separate Benutzung von Infusionsgeräten sind vorteilhafterweise Zusatzmodule als Stromquellen vorgesehen. Wenn ein solches Zusatzmodul an das Gehäuse des Infusionsgeräts angesteckt bzw. in das Gehäuse eingesteckt ist, ist das Infusionsgerät selbständig betriebsfähig, und zwar entweder in Verbindung mit einem Netzanschluß oder in Verbindung mit einem Akkumulator bzw. einer Batterie.

Die erfindungsgemäße Lösung hat den Vorteil, daß die Infusionsvorrichtung einen minimalen Platzbedarf hat. Ein Verkabelungsaufwand ist überhaupt nicht erforderlich. Die Plazierung der Infusionsgeräte ist wählbar und die Infusionsvorrichtung ist je nach Krankheitsbild und Krankheitsentwicklung des Patienten flexibel ausbaubar.

Im folgenden werden Ausführungsbeispiele der Erfindung unter Bezugnahme auf die Zeichnungen näher erläutert.

Es zeigen:
- Fig. 1: eine perspektivische Darstellung eines Geräteträgers,
- Fig. 2: den Geräteträger nach Fig. 1 nach Bestückung mit einigen Infusionsgeräten,
- Fig. 3: eine andere Ausführungsform des Geräteträgers in hängender Anordnung,
- Fig. 4: ein Infusionsgerät mit Zusatzmodul,
- Fig. 5: ein weiteres Infusionsgerät mit Zusatzmodul,
- Fig. 6: eine Ladestation für Zusatzmodule,
- Fig. 7: einen Geräteträger, bei dem die Säule aus auswechselbaren plattenförmigen Gehäusen besteht,
- Fig. 8: eine Seitenansicht von Fig. 7, jedoch mit anderer Gerätebestückung und
- Fig. 9: eine ähnliche Infusionsvorrichtung wie Fign. 7 und 8, jedoch mit anderer Gerätebestückung.

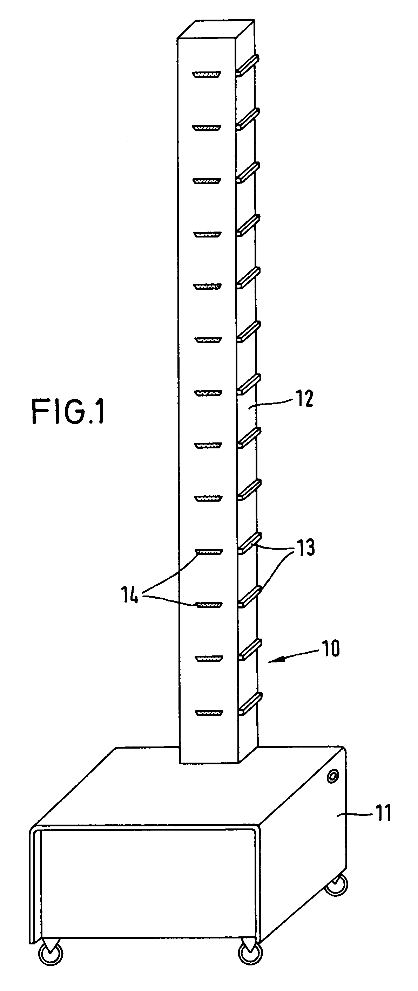

Der in Fig. 1 dargestellte Geräteträger 10 weist einen Sockel 11 in Form eines auf Rädern fahrbaren Wagens auf. Der Sockel 11 ist ein Gehäuse, das eine elektronische Überwachungs- und/oder Steuereinrichtung enthält. Von dem Sockel 11 ragt eine rechteckige Säule 12 auf, die in unterschiedlichen Höhen an den einander gegenüberliegenden Seitenwänden mit in regelmäßigen Abständen angeordneten Tragelementen 13 versehen ist, welche als leistenförmige Schiebeführungen ausgebildet sind. An der Frontseite der Säule 12 ist für jedes Paar von Tragelementen 13 ein vielkanaliges Kopplungselement 14, z.B. ein vielpoliger Steckverbinder vorgesehen.

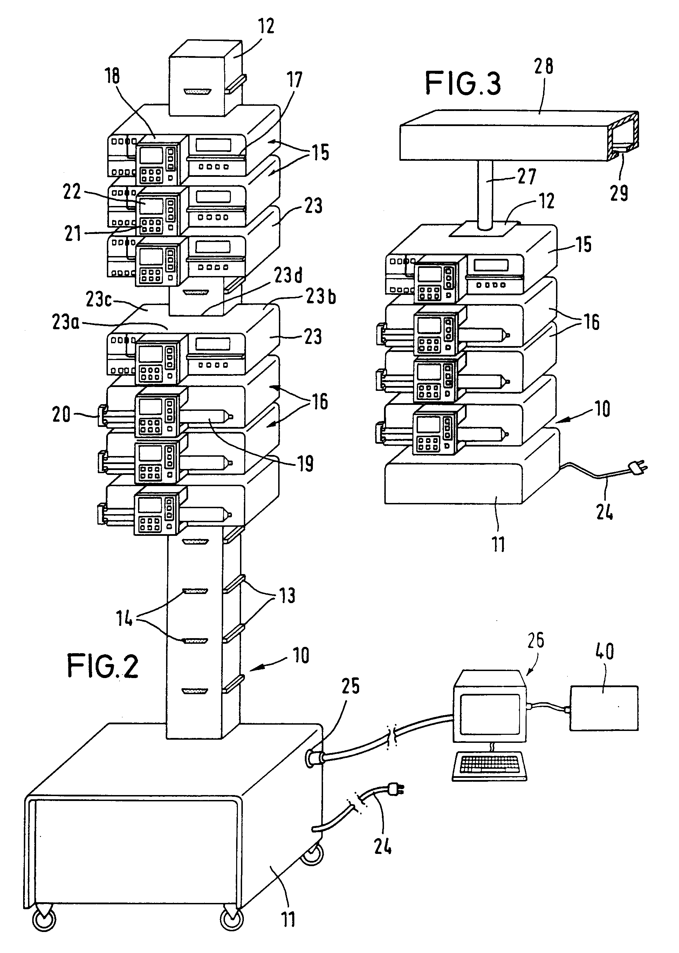

Gemäß Fig. 2 können an die Säule 12 Infusionsgeräte 15 oder 16 angesteckt werden. Bei dem vorliegenden Ausführungsbeispiel sind zwei Typen von Infusionsgeräten 15,16 vorgesehen. Die Infusionsgeräte 15 sind Schlauchpumpen, bei denen die zu infundierende Flüssigkeit durch einen Schlauch 17 dem Patienten zugeführt wird, welcher in einem Pumpengehäuse 18 durch eine peristaltische Pumpe fortlaufend abgequetscht wird, um die Flüssigkeit in dem Schlauch vorzutreiben. Die Infusionsgeräte 16 sind Spritzenpumpen, an denen jeweils eine Injektionsspritze 19 befestigt wird, deren Spritzenkolben durch einen angetriebenen Schieber 20 vorgeschoben wird, um den Inhalt der Spritze in einen zum Patienten führenden (nicht dargestellten) Schlauch auszudrücken. Jedes der Infusionsgeräte 15,16 weist an der Frontseite einen Bedien- und Anzeigenteil auf. Jedes Infusionsgerät enthält außer einer Antriebseinrichtung eine Steuereinrichtung, eine Eingabeeinrichtung 21 und eine Anzeigeeinrichtung 22.

Die Infusionsgeräte 15 und 16 haben ein Gehäuse 23, das in Draufsicht U-förmig gestaltet ist, wobei die Basis 23a die Frontseite mit den Bedien- und Anzeigeninstrumenten bildet, während die Schenkel 23b und 23c von der Basis 23a nach hinten abstehen und eine rechteckige Ausnehmung 23d begrenzen. Wenn das Gehäuse 23 an die Säule 12 angesteckt ist, füllt die Säule 12 die Ausnehmung 23d aus. An den Innenseiten der Schenkel 23b und 23c befinden sich nutartige Führungselemente, in die die leistenförmigen Tragelemente 13 der Säule 12 eingreifen.

An der Innenseite der Basis 23a des Gehäuses 23 ist ein mit dem Kopplungselement 14 zusammenpassendes Gegenstück als Steckverbinder vorgesehen. Wenn das Gehäuse 23 horizontal auf die senkrecht stehende Säule 12 aufgeschoben wird, kommt das gehäuseseitige Gegenstück mit dem Kopplungselement 14 in Eingriff, so daß eine mehrpolige Steckverbindung hergestellt wird.

Die Pole der Kopplungselemente 14 sind mit im Inneren der Säule 12 verlaufenden Kabeln verbunden. Zu diesen Kabeln gehören Stromversorgungs- und Datenleitungen. Die Stromversorgung der einzelnen Infusionsgeräte erfolgt durch den Sockel 11. Zu diesem Zweck ist der Sockel 11 mit einem Netzkabel 24 versehen.

Der Sockel 11 weist ferner einen Datenanschluß 25 auf, der mit einem externen Rechner 26 verbunden werden kann. Der Rechner 26 kann die im Sockel 11 aufbereiteten und über ein Bedienmodul 40 mit Bildschirm ergänzten Daten abfragen und mit den Daten weiterer medizinisch technischer Geräte zu einem Behandlungsprotokoll zusammenführen. Der Infusionsverlauf kann über das Bedienmodul 40 beobachtet werden.

Der Geräteträger 10 kann neben dem Krankenbett eines Patienten aufgestellt werden. Die für die Therapie benötigten Infusionsgeräte 15,16 werden von vorne auf die Säule 12 aufgeschoben, so daß sie diese Säule seitlich umgreifen. Danach kann entweder an den Infusionsgeräten am Rechner 26 oder am Bedienmodul 40 die Programmierung des Betriebs der Infusionsgeräte erfolgen.

Während bei dem Ausführungsbeispiel nach den Fign. 1 und 2 der Geräteträger eine große Anzahl (mehr als 10) Infusionsgeräte aufnehmen kann, zeigt Fig. 3 eine Ausführungsform, bei der der Geräteträger 10 in ähnlicher Weise ausgebildet ist, jedoch nur vier Infusionsgeräte 15,16 aufnehmen kann. Die Säule 12 ist mit einer Aufhängevorrichtung 27 versehen, welche an einer Wandschiene 28 des Krankenzimmers aufgehängt ist. Die Wandschiene 28 ist eine Profilschiene mit einem längslaufenden Führungsschlitz 29, durch den die Aufhängevorrichtung 27 hindurchragt und an dem sie mit einem (nicht dargestellten) Knebelverschluß arretierbar ist. Die Infusionsvorrichtung kann entlang des Schlitzes 29 verschoben und in unterschiedlichen Stellungen fixiert werden. Der Sockel 11 hängt bei diesem Ausführungsbeispiel an der Säule 12 und er befindet sich im Abstand über dem Boden, so daß im Bodenbereich ein Freiraum vorhanden ist, der für Großgeräte, wie z.B. Beatmer, benutzt werden kann.

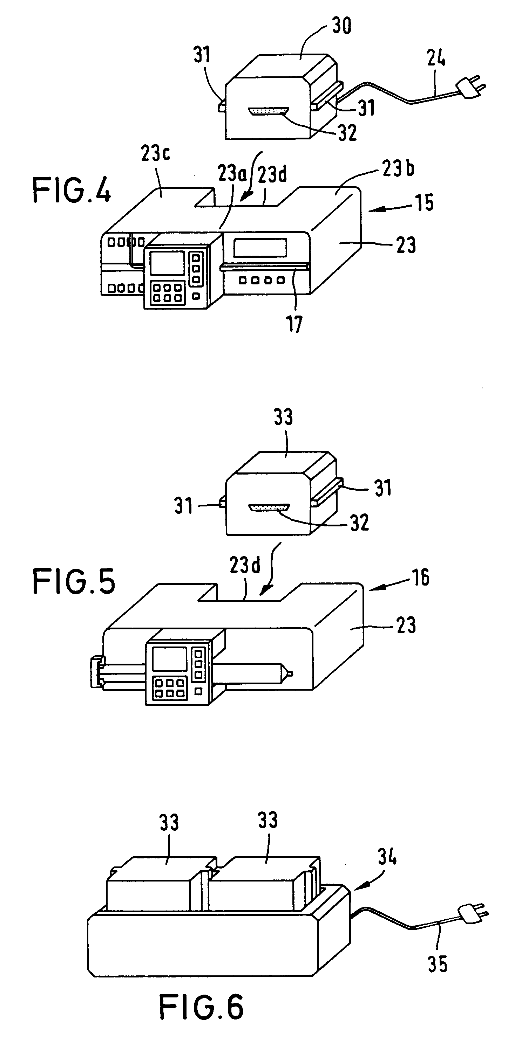

Fig. 4 zeigt ein Infusionsgerät 15, das von dem Geräteträger 10 gelöst ist und separat betrieben wird. In die Ausnehmung 23d ist ein Zusatzmodul 30 eingesetzt, das als Netzteil ausgebildet ist und ein Netzkabel 24 aufweist. Das Zusatzmodul 30 füllt die Ausnehmung 23d vollständig aus. Es ist an seinen Seiten mit leistenförmigen Tragelementen 31 versehen, die in gleicher Weise ausgebildet sind wie die Tragelemente 13 der Säule 12. An seiner der Basis 23a des Gehäuses 23 zugewandten Vorderseite weist es einen mehrpoligen Steckverbinder 32 auf, der in gleicher Weise ausgebildet ist wie die Steckverbinder 14 der Säule. Durch Einstecken des Zusatzmoduls 30 wird das Infusionsgerät 15 komplettiert, so daß es dann als selbständiges Gerät über das Netzkabel 24 an das Versorgungsnetz angeschlossen werden kann und selbständig betreibbar ist.

Fig. 5 zeigt ein Ausführungsbeispiel eines Infusionsgerätes, bei dem ein in die Ausnehmung 23d einsetzbares Zusatzmodul 33 in Form eines wiederaufladbaren Akkumulators vorgesehen ist. Das Gehäuse des Zusatzmoduls 33 ist genauso ausgebildet wie dasjenige des Zusatzmoduls 30, jedoch ist kein Netzkabel vorhanden.

Zum Wiederaufladen der Zusatzmodule 33 werden diese in eine Ladestation 34 eingestellt, wobei ihre Steckverbinder 32 mit entsprechenden Gegenstücken im Boden der Ladestation 34 verbunden werden. Die Ladestation 34 wird mit einem Netzkabel 35 an das Versorgungsnetz angeschlossen. Sie enthält ein Ladegerät zum Aufladen der Akkumulatoren der Zusatzmodule 33.

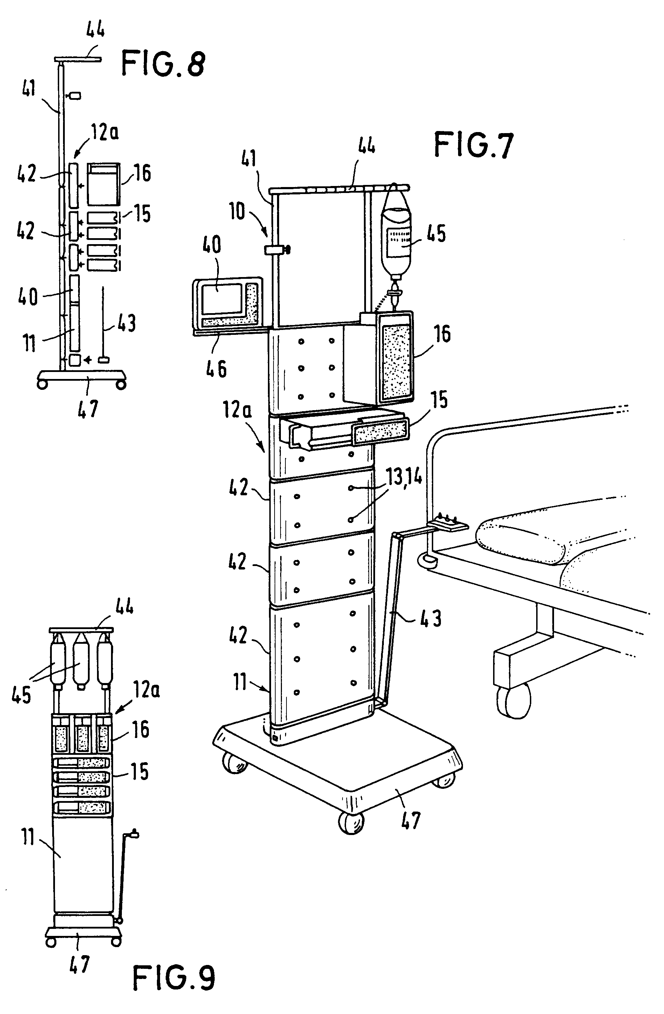

Die Fign. 7, 8 und 9 zeigen eine modifizierte Ausführungsform des Stecksystems für Infusionsgeräte und andere Zusatzmodule zur Unterstützung der Intensivtherapie. Die Säule 12a besteht hierbei aus einem Traggestell 41, das von einem fahrbaren Wagen 47 aufragt und an dem plattenförmige Gehäuse 42 in einer gemeinsamen Ebene befestigt sind. Das Traggestell 41 ist als Doppelrohrgestell mit zwei paralleln vertikalen Rohren ausgebildet. An diesen Rohren sind die Gehäuse 42 auswechselbar befestigt. Das untere Gehäuse 42 entspricht dem Sockel 11 der vorherigen Ausführungsbeispiele. Die Säule 12a ist eine Plattensäule oder Flachsäule, an die von der Vorderseite her die Infusionsgeräte 15 bzw. 16 angesteckt werden können. Zu diesem Zweck sind an jedem Gehäuse 42 Tragelemente 13 und Kopplungselemente 14 vorgesehen, an denen die Infusionsgeräte unter gleichzeitiger Herstellung der Versorgungs- und Datenverbindungen leicht lösbar befestigt werden können. Die Gehäuse 42 lassen sich an dem Traggestell 41 von der Frontseite her aufstecken. Der Anwender kann somit die Zusammensetzung und die Größe der Säule 12a selbst bestimmen. Die Infusionsgeräte 15,16 verfügen nicht über eine U-förmige Gehäusegestaltung, sondern sie sind an ihren ebenen Rückseiten mit Rastelementen ausgestattet, die mit den Rastelementen 13 der Gehäuse 42 zusammengreifen. Die Infusionsgeräte sind jeweils mit einem Netzteil und einer Batterie ausgestattet, so daß sie nach Abnahme von der Säule 12a unabhängig als Einzelpumpen betreibbar sind.

Die Infusionsvorrichtung muß nicht notwendigerweise eine selbständige fahrbare Einrichtung sein, sondern sie kann auch am Bettgestell angebracht sein, z.B. an der Fußwand des Patientenbettes.

An dem Traggestell 41 ist ein Halter 44 für Infusionsflaschen 45 vorgesehen. Von diesen Infusionsflaschen 45 führt jeweils ein Infusionsschlauch über ein Infusionsgerät 16 zum Patienten.

Wie Fig. 7 zeigt, ist das mit einem Bildschirm versehene Zusatzmodul 40 auf einen Ausleger 46 gestellt, der von dem Traggestell 41 absteht bzw. an diesem befestigt ist. Das Traggestell kann außer den plattenförmigen Gehäusen 42 noch als Träger für weitere Zusatzeinheiten zur Unterstützung der Intensivtherapie dienen.

## Patentansprüche

1. Infusionsvorrichtung mit einem als elektrische Versorgungseinheit ausgebildeten Geräteträger (10) und mehreren daran anbringbaren, elektrisch betriebenen Infusionsgeräten (15,16),
**dadurch gekennzeichnet,**
daß die Infusionsgeräte (15,16) am Geräteträger (18) als aufsteckbare Einheiten zu befestigen sind und daß der Geräteträger mindestens ein Kopplungselement (14) aufweist, das bei der Befestigung eines Infusionsgerätes am Geräteträger mit einem Gegenstück am Gehäuse (23) des Infusionsgerätes zu dessen Stromversorgung und/oder Datenanschluß zusammenwirkt.

2. Infusionsvorrichtung nach Anspruch 1, dadurch gekennzeichnet, daß Kopplungselemente (14) vorgesehen sind, die bei Befestigung eines Infusionsgerätes am Geräteträger Datenleitungen des Geräteträgers (10) mit Datenanschlüssen des Infusionsgerätes (15,16) koppeln.

3. Infusionsvorrichtung nach Anspruch 1 oder 2, dadurch gekennzeichnet, daß der Geräteträger (10) eine Säule (12,12a) aufweist, die in unterschiedlichen Höhen Tragelemente (13) zum Aufschieben von Infusionsgeräten (15,16) und jeweils mindestens ein Kopplungselement (14) aufweist.

4. Infusionsvorrichtung nach Anspruch 3, dadurch gekennzeichnet, daß die Säule (12a) aus einem Traggestell (41) mit auswechselbaren plattenförmigen Gehäusen (42) besteht, an denen die Infusionsgeräte (15,16) leicht lösbar zu befestigen sind.

5. Infusionsvorrichtung nach Anspruch 3, dadurch gekennzeichnet, daß die Tragelemente (13) aus Schiebeführungen bestehen und daß die Gehäuse (23) U-förmig gestaltet sind und an ihren Schenkeln (23b,23c) Führungselemente aufweisen, die mit den Tragelementen (13) zusammengreifen.

6. Infusionsvorrichtung nach einem der Ansprüche 1 bis 5, dadurch gekennzeichnet, daß Zusatzmodule (30,33) als Stromquellen vorgesehen sind, die in Ausnehmungen (23d) der Gehäuse (23) der Infusionsgeräte hineinpassen und einen Steckverbinder (32) sowie Tragelemente (31) aufweisen, die mit Führungselementen des Infusionsgerätes zusammengreifen.

7. Infusionsvorrichtung nach einem der Ansprüche 2 bis 6, dadurch gekennzeichnet, daß ein an den Geräteträger (10) angeschlossener oder anschließbarer Rechner (26) für die Dokumentation und/oder Steuerung des Betriebs der Infusionspumpen (15,16) vorgesehen ist.

8. Infusionsvorrichtung nach einem der Ansprüche 1 bis 7, dadurch gekennzeichnet, daß der Geräteträger (10) eine Aufhängevorrichtung (27) für die Befestigung an einer Wandschiene (28) aufweist.

9. Infusionsvorrichtung nach einem der Ansprüche 1 bis 8, dadurch gekennzeichnet, daß der Geräteträger (40) aus einem plattenförmigen Gehäuse besteht, das Steckverbinder (41) für die Infusionspumpen (42) aufweist.

## Claims

1. An infusion device with an apparatus support (10) designed as an electric supply unit and with a plurality of electrically operated infusion apparatuses (15, 16) attachable thereto,
characterised in
that said infusion apparatuses (15, 16) are attachable to said apparatus support (10) as push-on units and that said apparatus support has at least one coupling element (14) which, when an infusion apparatus is fastened to said apparatus support, cooperates with a counterpart at the housing (23) of said infusion apparatus to effect the current supply and/or the data connection thereto.

2. The infusion device of claim 1, characterised in that coupling elements (14) are provided which couple data lines of said apparatus support (10) to data connections of said infusion apparatus (15, 16) when an infusion apparatus is fastened to said apparatus support.

3. The infusion device of claim 1 or 2, characterised in that said apparatus support (10) has a column (12, 12a) with support elements (13) arranged at different heights for pushing infusion apparatus (15, 16) thereon and with at least one respective coupling element (14).

4. The infusion device of claim 3, characterised in that said column (12a) consists of a support structure (41) with exchangeable plate-shaped housings (42) at which said infusion apparatus (15, 16) may be attached for easy detachment.

5. The infusion device of claim 3, characterised in that said support elements (13) consist of slide guides and that said housings (23) are of U-shape with guide elements on their legs (23b, 23c) cooperating with said support elements (13).

6. The infusion device of one of claims 1 to 5, characterised in that additional modules (30, 33) are provided as power sources fitting into recesses (23d) of said housings (23) of said infusion apparatus and having a plug-in connector (32) as well as support elements (31) cooperating with support elements of said infusion apparatus.

7. The infusion device of one of claims 2 to 6, characterised in that a computer (26), which is connected to said apparatus support (10) or may be connected thereto, is provided for documentation and/or control of the operation of the infusion pumps (15, 16).

8. The infusion device of one of claims 1 to 7, characterised in that said apparatus support (10) has a suspension device (27) for fastening to a wall rail (28).

9. The infusion device of one of claims 1 to 8, characterised in that said apparatus support (40) consists of a plate-shaped housing with plug-in connectors (41) for the infusion pumps (42).

## Revendications

1. Dispositif de perfusion, avec un support d'appareils (10) réalisé sous forme d'unité d'alimentation électrique et plusieurs appareils de perfusion (15, 16) pouvant lui être adjoints, exploités électriquement, caractérisé en ce que les appareils de perfusions (15, 16) sont à fixer au support d'appareils (18) sous forme d'unités embrochables et que le support d'appareils présente au moins un élément de couplage (14), qui coopère, lors de la fixation d'un appareil de perfusion au support d'appareils, avec une pièce conjuguée située au carter (23) de l'appareil de perfusion, à son alimentation électrique et/ou sont raccordement pour transmission de données.

2. Dispositif de perfusion selon la revendication 1, caractérisé en ce que sont prévus des éléments de couplage (14), qui, lors de la fixation d'un appareil de perfusion au support d'appareils, produisent le couplage de lignes de transmission de données du support d'appareils (10) avec des raccordements pour données de l'appareil de perfusion (15, 16).

3. Dispositif de perfusion selon la revendication 1 ou 2, caractérisé en ce que le support d'appareils (10) présente une colonne (12, 12a), qui présente des éléments de support (13) à des hauteurs différentes, pour enfiler dessus des appareils de perfusion (15, 16) et, chaque fois, au moins un élément de couplage (14).

4. Dispositif de perfusion selon la revendication 3, caractérisé en ce que la colonne (12a) est composée d'un bâti support (41), comportant des boîtiers (42) échangeables, se présentant sous forme de plaques, auxquelles les appareils de perfusion (15, 16) sont facilement amovibles.

5. Dispositif de perfusion selon la revendication 3, caractérisé en ce que les éléments support (13) sont composés de guidage coulissant et que les carters (23) sont configurés en U et présentent, à leurs branches (23b, 23c), des éléments de guidage venant s'engager avec les éléments de support (13).

6. Dispositif de perfusion selon l'une des revendications 1 à 5, caractérisé en ce que les modules supplémentaires (30, 33) sont prévus comme sources de courant et s'ajustent dans des évidement (23d) du carter (23) des appareils de perfusion et présentent un connecteur (32), ainsi que des éléments de support (31), venant s'engager avec des éléments de guidage de l'appareil de perfusion.

7. Dispositif de perfusion selon l'une des revendications 2 à 6, caractérisé en ce qu'un calculateur (26), raccordé ou pouvant être raccordé aux supports d'appareils (10), est prévu, pour la documentation et/ou la commande de l'exploitation des pompes de perfusion (15, 16).

8. Dispositif de perfusion selon l'une des revendications 1 à 7, caractérisé en ce que le support d'appareils (10) présente un dispositif de suspension (27) pour la fixation à un rail mural (28).

9. Dispositif de perfusion selon l'une des revendications 1 à 8, caractérisé en ce que le support d'appareils (40) consiste en un carter en forme de plaque, qui présente des connecteurs (41) pour les pompes de perfusion.
